# EUROPEAN PATENT SPECIFICATION

(11) **EP 2 786 735 B1**
(45) Date of publication and mention of the grant of the patent: **17.05.2017**
(21) Application number: 12854332.9
(22) Date of filing: 29.11.2012
(51) Int. Cl.: A61K 8/06, A61K 8/27, A61K 8/29, A61K 8/41, A61K 8/49, A61K 8/81, A61Q 1/00, A61Q 17/04, A61K 8/34, A61K 8/02, A61Q 1/02, A61Q 5/02, A61Q 15/00, A61Q 19/10

(54) **OIL-IN-WATER-TYPE EMULSION COSMETIC**
ÖL-IN-WASSER-EMULSIONSKOSMETIKUM
COSMÉTIQUE D'ÉMULSION DE TYPE HUILE DANS L'EAU

(30) Priority: 30.11.2011 JP 2011261301
(43) Date of publication of application: 08.10.2014
(73) Proprietor: Kao Corporation, Chuo-Ku Tokyo 103-8210 (JP)
(72) Inventor: KODASHIMA, Hideki, Odawara-shi Kanagawa 250-0002 (JP)
(74) Representative: Hoffmann Eitle
(86) International application number: PCT/JP2012/080986
(87) International publication number: WO 2013/081073

(56) References cited:
- EP-A2- 1 310 237
- EP-A2- 1 310 237
- WO-A1-2011/044015
- WO-A1-2011/055761
- WO-A1-2011/065439
- WO-A1-2011/136011
- WO-A1-2011/136011
- WO-A2-2011/129291
- JP-A- 2003 104 859
- JP-A- 2003 104 859
- JP-A- 2011 046 670

## Description

### Field of the Invention

The present invention relates to an oil-in-water emulsified cosmetic composition having an excellent sunscreen effect.

### Background of the Invention

In recent years, the importance of measures for controlling sunburn in everyday life has been recognized, and accordingly also in the cosmetics having an ultraviolet protective effect, oil-in-water emulsion type sunscreen cosmetics which have a fresh feeling upon use and are easy to use continuously have been developed.

To enhance the ultraviolet protective effect, ultraviolet absorbers and metal oxide powders such as zinc oxide and titanium dioxide have been used in these oil-in-water emulsion cosmetics. However, the addition of an ultraviolet absorber in a large amount causes discoloration and results in the deterioration of feeling upon use such as poor spreadability and stickiness. Further, the addition of a metal oxide powder in a large amount not only causes powder aggregation, sedimentation, and the like, over time but also reduces long-term stability such as decreased viscosity, emulsion separation, and precipitation. When these are used in combination, the above problems are more likely to be caused.

To improve these problems, it has been proposed to use a combination of an ultraviolet absorber such as a dibenzoylmethane derivative, and silane and/or silicone-treated titanium dioxide (see Patent Literature 1). Also, there has been proposed an oil-in-water emulsion cosmetic which contains a water soluble polymer such as polyacrylic acid amide, xanthan gum, or sodium acrylate/acryloyldimethyl taurate copolymer (see Patent Literatures 2 and 3).

### Citation List

### Patent Literature

Patent Literature 1: JP-A-9-2929
Patent Literature 2: JP-A-2003-104859
Patent Literature 3: JP-A-2010-215602

WO 2011/055761 describes an oil-in-water emulsified cosmetic composition having a high UV-protective effect, long-term stability and an excellent feeling upon application. The composition comprises zinc oxide powder (A) having an average particle diameter of 0.1 to 1 µm, an average particle thickness of 0.01 to 0.2 µm, and an average aspect ratio of 3 or more and a polymer (B) selected from the group consisting of a polyacrylamide compound, a polyacrylic acid, and salts thereof.

EP 1310237 describes a light-protective cosmetic or dermatological composition, which comprises a hydroxybenzophenone or derivative; and an acrylamide (co)polymer or derivative.

WO 2011/136011 describes an O/W emulsion composition with excellent preparation stability, that combines an inorganic powder and an organic ultraviolet absorbent. The O/W emulsion composition is characterized by having the following first oil phase and second oil phase, which are separately dispersed in a water phase. The first oil phase contains an organic ultraviolet absorbent and has an average particle diameter of not more than 700 nm; and the second oil phase contains a silicone oil and has dispersed hydrophobized inorganic powder.

### Summary of the Invention

The present invention provides an oil-in-water emulsified cosmetic composition which comprises the following components (A) to (D) and satisfies the following conditions (i) and (ii):

### [Components]

(A) at least one selected from the group consisting of 2-(4-diethylamino-2-hydroxybenzoyl)benzoic acid hexyl ester, 2,4-bis{[4-(2-ethylhexyloxy)-2-hydroxy]phenyl}-6-(4-methoxyphenyl)-1,3,5-triazine, 2,4,6-tris[4-(2-ethylhexyloxycarbonyl)anilino]-1,3,5-triazine and 2-ethylhexyl dimethoxybenzylidene dioxoimidazolidine propionate,
(B) 8% by mass or more and 20% by mass or less of a liquid oil including a liquid organic ultraviolet absorber, the mass content ratio of the liquid organic ¹ultraviolet absorber in the liquid oil being 0.5 or more, wherein the liquid organic ultraviolet absorber is at least one selected from 2-ethylhexyl paramethoxycinnamate, octocrylene and dimethicodiethyl benzal malonate,
(C) a polyacrylamide compound, and
(D) a hydrophobized fine particle metal oxide powder;

### [Conditions]

(i) a total content of the component (A) and the component (D) ((A) + (D)) relative to a total amount of the cosmetic composition is 10% by mass or more, and
(ii) a content mass ratio of the total content of the component (A) and the component (D) ((A) + (D)) relative to the total amount of the liquid oil (component(B)) is 0.6 or more and 2 or less.

### Effects of the Invention

The oil-in-water emulsified cosmetic composition of the present invention has excellent long-term stability and a high ultraviolet protective effect. Further, the cosmetic composition has a good feeling upon use with smooth spreadability on the skin and controlled stickiness when applied, and extremely good waterproofness and perspiration resistance, whereby a high ultraviolet protective effect is anticipated in practical use.

### Brief Description of the Drawings

[Figure 1] Figure 1 is a photograph showing an emulsion state in which a powder enters the inner phase (oil phase) to form a nearly spherical good emulsion particle.
[Figure 2] Figure 2 is a photograph showing an emulsion state in which a powder enters the inner phase but the shape and size of the emulsion particle are nonuniform.
[Figure 3] Figure 3 is a photograph showing an emulsion state in which a powder leaks out to the outer phase.

### Embodiments for carrying out the Invention

In the oil-in-water emulsion composition described in the above Patent Literature 1, the discoloration of the oil-in-water emulsion composition is improved, but the high and low temperature stability are not satisfactory and the powder aggregation and sedimentation are caused, thereby sometimes posing the problem of the long-term stability.

Also, in the oil-in-water emulsion compositions described in the above Patent Literatures 2 and 3, the long-term stability of the oil-in-water emulsion compositions is improved, but the compositions have the problem of failing to achieve a sufficient ultraviolet protective effect despite the combination use of 4-tert-butyl-4'-methoxybenzoylmethane and 2-ethylhexyl paramethoxycinnamate as the organic ultraviolet absorbers and a hydrophobized fine particle metal oxide powder.

Under the circumstances, the present invention is to provide an oil-in-water emulsified cosmetic composition having an excellent ultraviolet protective effect, good long-term stability and a good feeling upon use by using an organic ultraviolet absorber and a hydrophobized fine particle metal oxide powder in combination.

Thus, the present inventor first investigated in detail the emulsion state of an oil-in-water emulsified cosmetic composition. As a result, the present inventor found that the high and low temperature stability are significantly reduced when a metal oxide powder leaks out to the outer phase (aqueous phase) of the oil-in-water emulsified cosmetic composition; and when an organic ultraviolet absorber which is solid at room temperature is used as the ultraviolet absorber with a metal oxide powder, the organic ultraviolet absorber which is solid at room temperature is likely to recrystallize in the inner phase (oil phase) due to the influence of the metal oxide powder, whereby a sufficient ultraviolet protective ability is not achieved.

These problems are likely to be improved by adding a large amount of a liquid oil capable of dissolving and dispersing the organic ultraviolet absorbers and the metal oxide powders, but a sticky feeling and a friction feeling are intensified due to the large amount of the liquid oil added, and it is further revealed that the waterproofness and perspiration resistance are affected, resulting in reduced ultraviolet protective effect (durability) in practical use, whereby a new problem is raised.

Consequently, to solve the above problems, the present inventor carried out various studies aiming at further improving ultraviolet protective ability in practical use while controlling the addition of a liquid oil.

In the case of using a solid organic ultraviolet absorber and a hydrophobized fine particle metal oxide powder in combination, a small amount of the liquid oil added typically tended to reduce the long-term stability. However, the present inventor found that when a specific solid organic ultraviolet absorber and a hydrophobized fine particle metal oxide powder are used in a predetermined amount or more together with a polyacrylamide compound and the content mass ratio thereof relative to the liquid oil is increased, an oil-in-water emulsified cosmetic composition having an excellent ultraviolet protective ability and extremely good waterproofness and perspiration resistance, together with high long-term stability, is obtained. Further, the present inventor found that such an oil-in-water emulsified cosmetic composition has a good feeling upon use with smooth spreadability and controlled stickiness, whereby the present invention was accomplished.

Accordingly, the present invention is an oil-in-water emulsified cosmetic composition which comprises the following components (A) to (D) and satisfies the following conditions (i) and (ii):

### [Components]

(A) at least one selected from the group consisting of 2-(4-diethylamino-2-hydroxybenzoyl)benzoic acid hexyl ester, 2,4-bis{[4-(2-ethylhexyloxy)-2-hydroxy]phenyl}-6-(4-methoxyphenyl)-1,3,5-triazine, 2,4,6-tris[4-(2-ethylhexyloxycarbonyl)anilino]-1,3,5-triazine and 2-ethylhexyl dimethoxybenzylidene dioxoimidazolidine propionate,
(B) 8% by mass or more and 20% by mass or less of a liquid oil including a liquid organic ultraviolet absorber, the mass content ratio of the liquid organic ultraviolet absorber in the liquid oil being 0.5 or more, wherein the liquid organic ultraviolet absorber is at least one selected from 2-ethylhexyl paramethoxycinnamate, octocrylene and dimethicodiethyl benzal malonate,
(C) a polyacrylamide compound, and
(D) a hydrophobized fine particle metal oxide powder;

### [Conditions]

(i) a total content of the component (A) and the component (D) ((A) + (D)) relative to a total amount of the cosmetic composition is 10% by mass or more, and
(ii) a content mass ratio of the total content of the component (A) and the component (D) ((A) + (D)) relative to the total amount of the liquid oil (component(B)) is 0.6 or more and 2 or less.

In the present invention, the term "solid state" refers to a state that does not have fluidity in the conditions of 1 atmospheric pressure at 25°C; in other words, a state under the temperature condition of less than a melting point (for amorphous substances which have no melting point, the term refers to a state in the temperature condition of less than a fusing point).

Further, the term "liquid state" refers to a state of matter that has fluidity in the conditions of 1 atmospheric pressure at 25°C; in other words, a state under the temperature condition of a melting point or more (for amorphous substances which have no melting point, the term refers to a state in the temperature condition of a fusing point or more).

The component (A) used in the present invention is a solid organic ultraviolet absorber and at least one selected from the group consisting of 2-(4-diethylamino-2-hydroxybenzoyl)benzoic acid hexyl ester, 2,4-bis{[4-(2-ethylhexyloxy)-2-hydroxy]phenyl}-6-(4-methoxyphenyl)-1,3,5-triazine, 2,4,6-tris[4-(2-ethylhexyloxycarbonyl)anilino]-1,3,5-triazine and 2-ethylhexyl dimethoxybenzylidene dioxoimidazolidine propionate.

These components are commercially available and examples include UVINUL A PLUS (2-(4-diethylamino-2-hydroxybenzoyl)benzoic acid hexyl ester; manufactured by BASF), TINOSORB S (2,4-bis{[4-(2-ethylhexyloxy)-2-hydroxy]phenyl}-6-(4-methoxyphenyl)-1,3,5-triazine; manufactured by BASF), UVINUL T-150 (2,4,6-tris[4-(2-ethylhexyloxycarbonyl)anilino]-1,3,5-triazine; manufactured by BASF), and Soft Shade DH (2-ethylhexyl dimethoxybenzylidene dioxoimidazolidine propionate; manufactured by Ajinomoto Co., Inc.).

The content of the component (A) relative to the total amount of the cosmetic composition is preferably 0.5% by mass or more, more preferably 0.8% by mass or more, even more preferably 1% by mass or more, and preferably 8% by mass or less, more preferably 7% by mass or less, even more preferably 5% by mass or less. Specifically, it is preferably 0.5 to 8% by mass, more preferably 0.8 to 7% by mass, even more preferably 1 to 5% by mass. When the content is within the range, a good ultraviolet protective effect is achieved and the stability and feeling upon use are also good.

The liquid oil (B) used in the present invention is an oil agent having fluidity at 25°C under 1 atmospheric pressure as mentioned above, and encompasses liquid organic ultraviolet absorbers and oil agents conventionally used in cosmetics. In the present invention, a liquid organic ultraviolet absorber is contained to enhance the ultraviolet protective effect.

As the liquid ultraviolet absorber, at least one selected from 2-ethylhexyl paramethoxycinnamate, octocrylene and dimethicodiethyl benzal malonate is used from the viewpoint of improving the emulsion state and long-term stability; 2-ethylhexyl paramethoxycinnamate is preferable.

The oil agent other than the above organic ultraviolet absorbers contained in the liquid oil is not particularly limited as long as it is in the liquid state under the condition of 1 atmospheric pressure at 25°C. Specific examples include hydrocarbon oils such as α-olefin oligomer, liquid isoparaffin, liquid paraffin, and squalane; triglycerides such as glyceryl trioctanoate, avocado oil, olive oil, sesame oil, rice bran oil, safflower oil, soybean oil, corn oil, rapeseed oil, castor oil, cotton seed oil, and mink oil; fatty acids such as oleic acid and isostearic acid; ester oils such as isopropyl myristate, butyl myristate, isopropyl palmitate, ethyl oleate, ethyl linoleate, isopropyl linoleate, cetyl caprylate, hexyl laurate, decyl myristate, decyl oleate, oleyl oleate, isostearyl laurate, isotridecyl myristate, isocetyl myristate, isostearyl myristate, octyldodecyl myristate, octyl palmitate, isocetyl palmitate, isostearyl palmitate, propylene glycol dioleate, isodecyl oleate, isopropyl isostearate, cetyl 2-ethylhexanoate, stearyl 2-ethylhexanoate, propylene glycol dicaprate, propylene glycol dioleate, glyceryl tri(2-ethylhexanoate), caprylic/capric triglyceride, isononyl isononanoate, diisopropyl sebacate, and propylene glycol isostearate; branched or unsaturated higher alcohols such as 2-octyldodecanol, isostearyl alcohol, and oleyl alcohol; and dimethyl polysiloxane. Of these, octyldodecyl myristate, isocetyl myristate, cetyl 2-ethylhexanoate, glyceryl tri(2-ethylhexanoate), caprylic/capric triglyceride, isononyl isononanoate, diisopropyl sebacate, propylene glycol isostearate, isohexadecane, squalane and hydrogenated polyisobutene are preferable.

The content of the component (B) relative to the total amount of the cosmetic composition is, from the viewpoints of controlling the stickiness and retaining the ultraviolet protective effec, suitably 8% by mass or more and 20% by mass or less. Further, the content of the component (B) is preferably 9% by mass or more, more preferably 10% by mass or more, and preferably 20% by mass or less, more preferably 18% by mass or less. Specifically, it is 8 to 20% by mass, preferably 9 to 20% by mass, more preferably 10 to 20% by mass, even more preferably 10 to 18% by mass.

The mass content ratio of the liquid organic ultraviolet absorber in the liquid oil is 0.5 or more, preferably 0.6 or more, more preferably 0.7 or more, to enhance the ultraviolet protective effect while reducing the liquid oil content.

The polyacrylamide compound (C) used in the present invention includes a polyacrylamide and an acrylamide copolymer, and examples of the acrylamide copolymer include copolymers containing acrylamide and/or acryloyldimethyl taurate as a constituent unit.

Examples of the copolymers containing acrylamide and/or acryloyldimethyl taurate as a constituent unit include a copolymer of hydroxyethyl acrylate and acryloyldimethyl taurate, a copolymer of acrylate and acryloyldimethyl taurate, and a copolymer of acrylamide and acrylate, and a copolymer of acrylic acid, acrylamide, acrylate, and acryloyldimethyl taurate.

These components are commercially available, and examples of the polyacrylamide include SEPIGEL 305 (polyacrylamide, hydrogenated polyisobutene (or (C13, 14)isoparaffin), laureth-7, water); examples of the copolymer of hydroxyethyl acrylate and acryloyldimethyl taurate include SEPINOV EMT 10 (hydroxyethyl acrylate/Na acryloyldimethyl taurate copolymer), SIMULGEL NS (hydroxyethyl acrylate/Na acryloyldimethyl taurate copolymer, squalane, polysorbate 60, water), SIMULGEL FL (hydroxyethyl acrylate/Na acryloyldimethyl taurate copolymer, isohexadecane, polysorbate 60, water), SEPIPLUS S (hydroxyethyl acrylate/Na acryloyldimethyl taurate copolymer, polyisobutene, PEG-7 trimethylolpropane coconut alkyl ether, water); examples of the copolymer of acrylate and acryloyldimethyl taurate include SIMULGEL EG (Na acrylate/Na acryloyldimethyl taurate copolymer, isohexadecane, polysorbate 80, water); examples of the copolymer of acrylamide and acrylate include SEPIPLUS 265 (acrylamide/ammonium acrylate copolymer, polyisobutene, polysorbate 20, water); and examples of the copolymer of acrylic acid, acrylamide, acrylate and acryloyldimethyl taurate include SEPIPLUS 400 (polyacrylate-13, polyisobutene, polysorbate 20, water).

Of these, the copolymer of acrylate and acryloyldimethyl taurate is preferable, with Na acrylate/Na acryloyldimethyl taurate copolymer being more preferable.

The content of the component (C) relative to the total amount of the cosmetic composition of the present invention is preferably 0.1% by mass or more, more preferably 0.2% by mass or more, even more preferably 0.3% by mass or more, from the viewpoint of smooth spreadability when applied. Further, it is preferably 2% by mass or less, more preferably 1.6% by mass or less, even more preferably 1.4% by mass or less. Specifically, it is preferably 0.1 to 2% by mass, more preferably 0.2 to 1.6% by mass, more preferably 0.2 to 1.4% by mass, even more preferably 0.3 to 1.4% by mass.

The hydrophobized fine particle metal oxide powder (D) used in the present invention is preferably one or two or more metal oxide powders selected from the group consisting of zinc oxide, titanium dioxide and cerium oxide, due to the high ultraviolet scattering effect. Further, these metal oxide powders may contain a trace metal having a valence of +2 or more, and metals such as iron, zirconium, calcium, manganese, magnesium, or yttrium may be contained in the above fine particle metal oxide powder singly or in a suitable combination of two or more.

The fine particle zinc oxide powder is commercially available and examples include FINEX-25, FINEX-50, FINEX-75 (manufactured by Sakai Chemical Industry Co., Ltd.), MZ500 series, MZ700 series (manufactured by TAYCA CORPORATION), and ZnO-350 (manufactured by Sumitomo Osaka Cement Co., Ltd.); and the flaky powder of zinc oxide described in JP-B-3073887 may be mentioned. Examples of the fine particle titanium dioxide powder include TTO-55 series, TTO-51 series (manufactured by ISHIHARA SANGYO KAISHA, LTD.), JR series, and JA series (manufactured by TAYCA CORPORATION), which are commercially available. Further, the fine particle cerium oxide includes high purity cerium available from Nikki Co., Ltd. or AGC Seimi Chemical Co., Ltd. Of these, it is preferable to use a fine particle zinc oxide powder or fine particle titanium dioxide powder.

The average particle diameter of the fine particle metal oxide powder used in the present invention is, from the viewpoint of preventing the powder aggregation, preferably 0.01 µm or more, more preferably 0.012 µm or more, even more preferably 0.015 µm or more. Also, from the viewpoint of preventing the preparation opacification, it is preferably 1 µm or less, more preferably 0.5 µm or less, even more preferably 0.4 µm or less. Specific average particle diameter is within the range of preferably 0.01 to 1 µm, more preferably 0.012 to 0.5 µm, even more preferably 0.015 to 0.4 µm. In addition, the average particle diameter is measured by the laser diffraction and scattering method.

Examples of the shape of the fine particle metal oxide powder used in the present invention include spherical, flaky, rod, spindle, needle, and indeterminate shapes, and any shape may be used as long as the average particle diameter is within the range mentioned above.

The hydrophobization treatment to the above fine particle metal oxide powder is not particularly limited, and various surface treatments may be carried out in advance such as fluorine compound treatment, silicone treatment, silicone resin treatment, pendant treatment, silane coupling agent treatment, titanium coupling agent treatment, oil agent treatment, N-acylated lysine treatment, polyacrylic acid treatment, metallic soap treatment, amino acid treatment, inorganic compound treatment, plasma treatment, mechanochemical treatment, and silane compound or silazane compound treatment.

Preferable examples include surface treatment using silicone or a silicone resin, treatment using methyl hydrogen polysiloxane or the methyl hydrogen polysiloxane-dimethylpolysiloxane copolymer represented by the following formula (1) as a surface treatment agent, and treatment using a silane compound or a silazane compound as a surface treatment agent. The surface treatment using silicone or a silicone resin and the surface treatment using methyl hydrogen polysiloxane are more preferable.

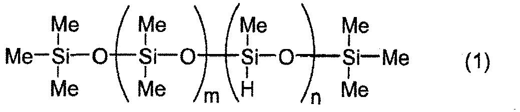

wherein m and n are each an integer, and 1 ≤ m + n ≤ 60.

Examples of the above surface treatment using silicone or a silicone resin include, as described in JP-B-3187440, a method including coating a metal oxide such as a zinc oxide powder in the non-gaseous phase state with at least one silicone compound containing organo polysiloxanes and a silicone resin (provided that silane compounds are excluded) and subsequently baking the metal oxide at a temperature of 600 to 950°C in an oxygen-containing atmosphere to coat the metal oxide surface with silicon oxide.

The above silane compound or silazane compound is preferably those having an alkyl or fluoroalkyl group having 1 to 20 carbon atoms and reactive to an inorganic oxide, and specifically is the silane compound represented by the following formula (2) or the silazane compound represented by the following formula (3). One or two or more of these may be used.

RR¹ₙSiX₃₋ₙ (2)

wherein n is an integer of 0 or 1, R represents an alkyl or fluoroalkyl group having 1 to 20 carbon atoms (which may be linear or branched), R¹ represents an alkyl group having 1 to 6 carbon atoms, and X represents a halogen atom or an alkoxy group, and

R²R³R⁴SiNHSiR⁵R⁶R⁷ (3)

wherein R² to R⁷ may be the same or different and each represent an alkyl or fluoroalkyl group having 1 to 20 carbon atoms (which may be linear or branched).

Specific examples of the silane compound include hexyltrimethoxysilane, octyltrimethoxysilane, decyltrimethoxysilane, octadecyltrimethoxysilane, octyltriethoxysilane, trifluoropropyltrimethoxysilane, and heptadecafluorodecyltrimethoxysilane. Of these, octyltriethoxysilane and octyltrimethoxysilane are preferable. Preferable examples of the silazane compound include hexamethyldisilazane. Such a silane compound or silazane compound is characterized in easily rendering the even treatment and being easily supplied at a low cost, and further the cosmetic composition containing the fine particle metal oxide powder (D) surface treated with these compounds is preferable because it has excellent properties such as dispersibility.

Examples of the treatment method using the aforementioned silane or silazane compound include a method including allowing a silane or silazane compound to undergo chemical reactions with a reactive group on the surface of a metal oxide such as zinc oxide, e.g., a method including mixing a silane or silazane compound and a metal oxide such as a zinc oxide powder in an organic solvent such as n-hexane, cyclohexane, and a lower alcohol, and performing pulverization, if necessarily, and then removing the organic solvent by heating or reducing pressure, and applying heat treatment preferably at 80 to 250°C.

Examples thereof also include a method which includes subjecting a cosmetic pigment to coating treatment with a specific polysiloxane compound, and then to surface treatment using alkylalkoxysilane in water as described in JP-A-2007-326902.

The amount of the surface treatment agent for coating the fine particle metal oxide powder is, relative to the total amount of powders to be used, preferably 3% by mass or more, more preferably 5% by mass or more, and preferably 15% by mass or less, more preferably 10% by mass or less. The specific amount of coating is preferably 3 to 15% by mass, more preferably 5 to 10% by mass. With the amount of coating within such a range, the surface treatment agent evenly coats the powder surface and is free from the aggregation and precipitation on the powder surface of zinc oxide or the like.

The content of the hydrophobized fine particle metal oxide (D) used in the present invention, relative to the total amount of the cosmetic composition, is preferably 8% by mass or more, more preferably 9% by mass or more, even more preferably 10% by mass or more, and preferably 25% by mass or less, more preferably 23% by mass or less, even more preferably 20% by mass or less. The specific content is preferably 8 to 25% by mass, more preferably 9 to 23% by mass, even more preferably 10 to 20% by mass. When the content is within the range, the powder dispersibility is good and thus a problem of a large increase in the viscosity of the preparation is avoided.

It is preferable that a hydrophobized fine particle zinc oxide powder, as the hydrophobized fine particle metal oxide (D), be contained in 60% by mass or more in the component (D) because it leads to the enhancement of the UVA protective ability and the improvement of high temperature stability.

It is preferable that the oil-in-water emulsified cosmetic composition of the present invention contain a saturated monohydric alcohol having 1 to 3 carbon atoms (E) from the viewpoints of spreadability when applied, control of a sticky feeling, long-term stability and waterproofness and perspiration resistance. Examples of the saturated monohydric alcohol having 1 to 3 carbon atoms (E) include methyl alcohol, ethyl alcohol, propyl alcohol, and isopropyl alcohol.

The content of the component (E) of the present invention is not particularly limited, but, from the viewpoints of enhancing the spreadability when applied, controlling a sticky feeling, and improving the long-term stability and waterproofness and perspiration resistance, is, relative to the total amount of the cosmetic composition, preferably 8% by mass or more, more preferably 10% by mass or more, even more preferably 12% by mass or more, and preferably 22% by mass or less, more preferably 20% by mass or less, even more preferably 18% by mass or less. The specific content is preferably 8 to 22% by mass, more preferably 10 to 20% by mass, even more preferably 12 to 18% by mass.

In the present invention, it is essential that the total content of the component (A) and the component (D) ((A) + (D)) relative to the total amount of the cosmetic composition be 10% by mass or more to achieve a good ultraviolet protective effect. The total content of (A + D) relative to the total amount of the cosmetic composition may be 10% by mass or more, but is more preferably 11% by mass or more, and preferably 28% by mass or less, more preferably 25% by mass or less, even more preferably 23% by mass or less. Specifically, it is preferably 10 to 28% by mass, more preferably 10 to 25% by mass, even more preferably 11 to 25% by mass, even more preferably 11 to 23% by mass.

Further, it is essential that the content mass ratio of the total content of the component (A) and the component (D) ((A) + (D)) relative to the total amount of liquid oil be 0.6 or more and 2 or less to achieve good long-term stability. The total amount of (A + D) relative to the total amount of liquid oil may be 0.6 or more and 2 or less, but is preferably 0.7 or more, more preferably 0.75 or more, and preferably 1.8 or less, more preferably 1.7 or less. Specifically, it is preferably 0.7 to 2, more preferably 0.7 to 1.8, even more preferably 0.75 to 1.8, even more preferably 0.75 to 1.7.

The oil-in-water emulsified cosmetic composition of the present invention may comprise a surfactant for the purpose of adjusting the feeling upon use and long-term stability. The surfactant is not particularly limited and any of nonionic surfactants, anionic surfactants, cationic surfactants, amphoteric surfactants, natural surfactants, polyether modified silicones, silicone-containing surfactants such as siloxane derivatives, and surfactants containing a perfluoroalkyl group may be used. One or two or more of these can be used.

Of these surfactants, nonionic surfactants, polyether modified silicones and silicone-containing surfactants are preferable, and specific examples include sorbitan monoisostearate, dimethicone copolyol, polyoxyethylene hydrogenated castor oil (40EO), polyoxyethylene castor oil (40EO), polyoxyethylene sorbitan monolaurate (20EO), polyoxyethylene monostearate (20EO), polyoxyethylene sorbitan oleate (20EO), polyoxyethylene (7) lauryl ether, and PEG-7 trimethylolpropane coconut ether.

The oil-in-water emulsified cosmetic composition of the present invention has good feeling upon use and long-term stability even when the above surfactant is not used in combination. When a surfactant is added in a large amount, a sticky feeling derived from the surfactant may sometimes be caused. Thus the preferable amount added relative to the total amount of the cosmetic composition is preferably 3% by mass or less, more preferably 1% by mass or less. The specific range is preferably 0 to 3% by mass, more preferably 0 to 1% by mass, even more preferably 0 to 0.5% by mass.

In the oil-in-water emulsified cosmetic composition of the present invention, the content of water relative to the total amount of the oil-in-water emulsified cosmetic composition is, from the viewpoint of forming the oil-in-water emulsified composition having excellent long-term stability, preferably 40% by mass or more, more preferably 45% by mass or more, and preferably 75% by mass or less, more preferably 70% by mass or less. Specifically, it is preferably 40 to 75% by mass, more preferably 45 to 70% by mass.

Furthermore, the oil-in-water emulsified cosmetic composition of the present invention may comprise, in accordance with the purpose, in addition to the above components, within the range in which the effect of the present invention is not impaired, higher alcohols, fatty acids, esters, sterols, fatty acid sterol esters, hydrocarbons, oils and fats, silicone oil, moisturizers, water soluble polymers other than the component (A) of the present invention, plant extracts, vitamins, antioxidants, antimicrobial preservatives, antiphlogistic agents, insect repellents, physiologically active components, salts, chelating agents, neutralizers, pH adjusting agents, fragrances, or the like.

The oil-in-water emulsified cosmetic composition of the present invention is suitably used as a hair cosmetic composition such as a shampoo, a hair rinse, and a hair conditioner; and a skin cosmetic composition such as a facial wash, a cleansing cosmetic composition, a sunscreen cosmetic composition, a facial pack, and a massage cosmetic composition. Of these, the oil-in-water emulsified cosmetic composition is more preferably applied to a sunscreen cosmetic composition (a lotion, a cream, an emulsion, an essence, and the like), a suntan, a makeup base cosmetic composition, and a foundation having a ultraviolet protective ability, and the like.

As for the embodiments described above, the present invention further discloses the following embodiments.
[1] An oil-in-water emulsified cosmetic composition comprising the following components (A) to (D), and satisfying the following conditions (i) and (ii):
   [Components]
      (A) at least one selected from the group consisting of 2-(4-diethylamino-2-hydroxybenzoyl)benzoic acid hexyl ester, 2,4-bis{[4-(2-ethylhexyloxy)-2-hydroxy]phenyl}-6-(4-methoxyphenyl)-1,3,5-triazine, 2,4,6-tris[4-(2-ethylhexyloxycarbonyl)anilino]-1,3,5-triazine and 2-ethylhexyl dimethoxybenzylidene dioxoimidazolidine propionate,
      (B) 8% by mass or more and 20% by mass or less of a liquid oil,
      (C) a polyacrylamide compound, and
      (D) a hydrophobized fine particle metal oxide powder;
   [Conditions]
      (i) a total content of the component (A) and the component (D) ((A) + (D)) relative to a total amount of the cosmetic composition is 10% by mass or more, and
      (ii) a content mass ratio of the total content of the component (A) and the component (D) ((A) + (D)) relative to the total amount of the liquid oil (component(B)) is 0.6 or more and 2 or less.
[2] The oil-in-water emulsified cosmetic composition according to [1], wherein the component (A) is preferably a solid organic ultraviolet absorber.
[3] The oil-in-water emulsified cosmetic composition according to [1] or [2], wherein the content of the component (A) relative to the total amount of the cosmetic composition is preferably 0.5% by mass or more, more preferably 0.8% by mass or more, even more preferably 1% by mass or more, and preferably 8% by mass or less, more preferably 7% by mass or less, even more preferably 5% by mass or less.
[4] The oil-in-water emulsified cosmetic composition according to [1] to [3], wherein the content of the component (A) relative to the total amount of the cosmetic composition is preferably 0.5 to 8% by mass, more preferably 0.8 to 7% by mass, even more preferably 1 to 5% by mass.
[5] The oil-in-water emulsified cosmetic composition according to [1] to [4], wherein the component (B) preferably comprises a liquid organic ultraviolet absorber and an oil agent for a cosmetic composition.
[6] The oil-in-water emulsified cosmetic composition according to [5], wherein the liquid organic ultraviolet absorber is preferably at least one selected from the group consisting of 2-ethylhexyl paramethoxycinnamate, 2-ethoxyethyl paramethoxycinnamate, isopropyl paramethoxycinnamate/diisopropyl cinnamic acid ester mixture, methylbis(trimethylsiloxy)silylisopentyl trimethoxycinnamate, amyl paradimethylaminobenzoate, 2-ethylhexyl paradimethylaminobenzoate, ethyleneglycol salicylate, 2-ethylhexyl salicylate, benzyl salicylate, homomenthyl salicylate, octocrylene and dimethicodiethyl benzal malonate.
[7] The oil-in-water emulsified cosmetic composition according to [5] or [6], wherein the oil agent for a cosmetic composition is preferably at least one selected from the group consisting of a hydrocarbon oil, a triglyceride, a fatty acid, an ester oil, a higher alcohol and dimethylpolysiloxane.
[8] The oil-in-water emulsified cosmetic composition according to [1] to [7], wherein the content of the component (B) relative to the total amount of the cosmetic composition is preferably 10% by mass or more, more preferably 9% by mass or more, even more preferably 8% by mass or more, and preferably 20% by mass or less, more preferably 18% by mass or less.
[9] The oil-in-water emulsified cosmetic composition according to [1] to [8], wherein the content of the component (B) relative to the total amount of the cosmetic composition is preferably 8 to 20% by mass, more preferably 9 to 20% by mass, even more preferably 10 to 20% by mass, even more preferably 10 to 18% by mass.
[10] The oil-in-water emulsified cosmetic composition according to [5] to [9], wherein the mass content ratio of the liquid organic ultraviolet absorber in the component (B) is preferably 0.5 or more, more preferably 0.6 or more, even more preferably 0.7 or more.
[11] The oil-in-water emulsified cosmetic composition according to [1] to [10], wherein the component (C) is preferably at least one selected from the group consisting of a polyacrylamide and a copolymer containing acrylamide and/or acryloyl dimethyl taurate as a constituent unit.
[12] The oil-in-water emulsified cosmetic composition according to [1] to [11], wherein the content of the component (C) relative to the total amount of the cosmetic composition is preferably 0.1% by mass or more, more preferably 0.2% by mass or more, even more preferably 0.3% by mass or more, and preferably 2% by mass or less, more preferably 1.6% by mass or less, even more preferably 1.4% by mass or less.
[13] The oil-in-water emulsified cosmetic composition according to [1] to [12], wherein the content of the component (C) relative to the total amount of the cosmetic composition is preferably 0.1 to 2% by mass, more preferably 0.2 to 1.6% by mass, even more preferably 0.2 to 1.4% by mass, even more preferably 0.3 to 1.4% by mass.
[14] The oil-in-water emulsified cosmetic composition according to [1] to [13], wherein the component (D) is preferably one or two or more hydrophobized metal oxide powders selected from the group consisiting of zinc oxide, titanium dioxide and cerium oxide.
[15] The oil-in-water emulsified cosmetic composition according to [1] to [14], wherein the average particle diameter of the component (D) is preferably 0.01 µm or more, more preferably 0.012 µm or more, even more preferably 0.015 µm or more, and preferably 1 µm or less, more preferably 0.5 µm or less, even more preferably 0.4 µm or less.
[16] The oil-in-water emulsified cosmetic composition according to [1] to [15], wherein the average particle diameter of the component (D) is preferably 0.01 to 1 µm, more preferably 0.012 to 0.5 µm, even more preferably 0.015 to 0.4 µm.
[17] The oil-in-water emulsified cosmetic composition according to [1] to [16], wherein a hydrophobization treatment to the fine particle metal oxide powder is preferably fluorine compound treatment, silicone treatment, silicone resin treatment, pendant treatment, silane coupling agent treatment, titanium coupling agent treatment, oil agent treatment, N-acylated lysine treatment, polyacrylic acid treatment, metallic soap treatment, amino acid treatment, inorganic compound treatment, plasma treatment, mechanochemical treatment, or silane compound or silazane compound surface treatment, more preferably at least one surface treatment selected from a surface treatment using silicone or a silicone resin and a surface treatment using methyl hydrogen polysiloxane.
[18] The oil-in-water emulsified cosmetic composition according to [1] to [17], wherein the amount of hydrophobization treatment of the component (D) relative to the total amount of the powder is preferably 3% by mass or more, more preferably 5% by mass or more, and preferably 15% by mass or less, more preferably 10% by mass or less.
[19] The oil-in-water emulsified cosmetic composition according to [1] to [18], wherein the content of the component (D) relative to the total amount of the cosmetic composition is preferably 8% by mass or more, more preferably 9% by mass or more, even more preferably 10% by mass or more, and preferably 25% by mass or less, more preferably 23% by mass or less, even more preferably 20% by mass or less.
[20] The oil-in-water emulsified cosmetic composition according to [1] to [19], wherein the content of the component (D) relative to the total amount of the cosmetic composition is preferably 8 to 25% by mass, more preferably 9 to 23% by mass, even more preferably 10 to 20% by mass.
[21] The oil-in-water emulsified cosmetic composition according to [1] to [20] further compsiring (E) a saturated monohydric alcohol having 1 to 3 carbon atoms.
[22] The oil-in-water emulsified cosmetic composition according to [1] to [21], wherein the content of the component (E) relative to the total amount of the cosmetic composition is preferably 8% by mass or more, more preferably 10% by mass or more, even more preferably 12% by mass or more, and preferably 22% by mass or less, more preferably 20% by mass or less, even more preferably 18% by mass or less.
[23] The oil-in-water emulsified cosmetic composition according to [1] to [22], wherein the content of the component (E) relative to the total amount of the cosmetic composition is preferably 8 to 22% by mass, more preferably 10 to 20% by mass, even more preferably 12 to 18% by mass.
[24] The oil-in-water emulsified cosmetic composition according to [1] to [23], wherein the total amount of (A + D) relative to the total amount of the cosmetic composition is preferably 10% by mass or more, more preferably 11% by mass or more, and preferably 28% by mass or less, more preferably 25% by mass or less, even more preferably 23% by mass or less.
[25] The oil-in-water emulsified cosmetic composition according to [1] to [24], wherein the total amount of (A + D) relative to the total amount of the cosmetic composition is preferably 10 to 28% by mass, more preferably 10 to 25% by mass, even more preferably 11 to 25% by mass, even more preferably 11 to 23% by mass.
[26] The oil-in-water emulsified cosmetic composition according to [1] to [25], wherein the total amount of (A + D) relative to the total amount of the liquid oil is preferably 0.7 or more, more preferably 0.75 or more, and preferably 1.8 or less, more preferably 1.7 or less.
[27] The oil-in-water emulsified cosmetic composition according to [1] to [26], wherein the total amount of (A + D) relative to the total amount of the liquid oil is preferably 0.7 to 2, more preferably 0.7 to 1.8, even more preferably 0.75 to 1.8, even more preferably 0.75 to 1.7.
[28] The oil-in-water emulsified cosmetic composition according to [1] to [27], comprising preferably 0 to 3% by mass, more preferably 0 to 1% by mass, even more preferably 0 to 0.5% by mass, of a surfactant.
[29] The oil-in-water emulsified cosmetic composition according to [1] to [28], wherein the content of water relative to the total amount of the cosmetic composition is preferably 40% by mass or more, more preferably 45% by mass or more, and preferably 75% by mass or less, more preferably 70% by mass or less.
[30] The oil-in-water emulsified cosmetic composition according to [1] to [29], which is used for a sunscreen application.

### Example 1

Hereinafter, the present invention is described in details with reference to Examples and Comparative Examples, but is not limited thereto.

### Examples 1 to 21, Comparative Examples 1 to 17

Oil-in-water emulsified cosmetic compositions having the formulations shown in Tables 1 to 4 were prepared in accordance with a routine method. Using these cosmetic compositions, (1) UV protection durability test, (2) emulsion state test, (3) long-term stability test, and (4) feeling upon use test shown below were carried out. The results are also shown in Tables 1 to 4.

### (1) UV Protective ability (Durability)

Each of the samples shown in Tables 1 to 4 was evenly applied onto a quartz glass plate so as to give 2 mg/cm² and dried for 15 minutes in the air. Subsequently, the quartz glass plate on which the sample was applied was irradiated with ultraviolet rays from a predetermined distance (10 mm). The transmitted ultraviolet rays at that time were detected using an SPF analyzer (Optometices) at 6 or more sites on the quartz glass plate in the range of 290 to 400 nm to obtain the averaged spectrum, thereby calculating the SPF values.

Then, the entire quartz glass plate on which the sample was applied was soaked in a water bath at 20°C with the applied surface being up, and water was lightly stirred for 15 minutes while the water temperature was maintained at 20°C. Subsequently, the quartz glass plate was slowly taken out, allowed to stand for 30 minutes or more to dry. The procedure was repeated twice and then SPF values were calculated again in the same method.

### [long-lasting effect evaluation of UV protective effect]

UV protective ability (long-lasting effect) (%) = (SPF value measured after soaking the quarts glass plate on which the sample was applied in the water bath) / (SPF value measured before soaking the quarts glass plate on which the sample was applied in the water bath) × 100

### [UV protective ability (long-lasting effect) evaluation criteria]

A: 80% or more
B: 70% or more and less than 80%
C: 50% or more and less than 70%
D: less than 50%

### (2) Emulsion state

The oil-in-water emulsified cosmetic composition immediately after production was observed under a microscope and evaluated based on the following criteria.

### [Judgment criteria for emulsion state]

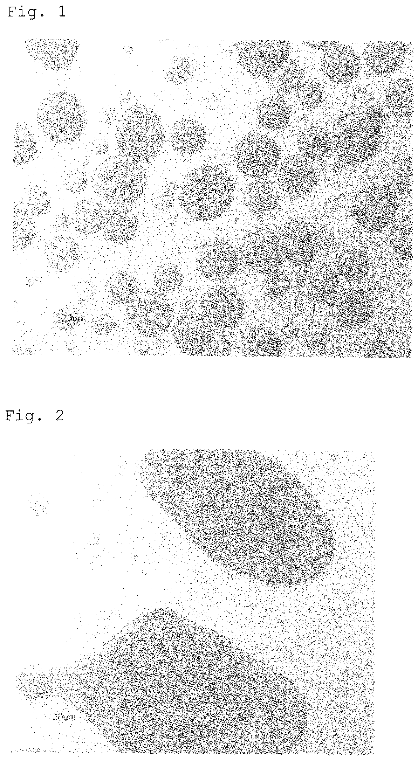

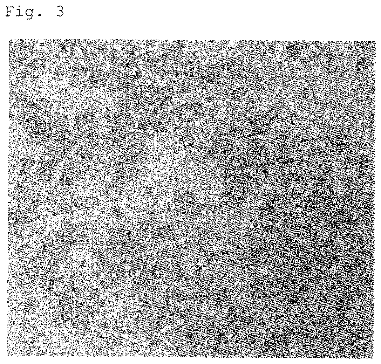

A: A powder enters the inner phase (oil phase) and a nearly spherical good emulsion particle is formed (Figure 1) .
B: A powder enters the inner phase but the shape and size of the emulsion particle are nonuniform (Figure 2).
C: A powder leaks out to the outer phase (aqueous phase) (Figure 3).

### [Judgment criteria for crystallization state]

A: No crystallization was found in the inner phase (oil phase)
C: Crystallization was found in a part of the inner phase

### (3) Long-term stability test

The oil-in-water emulsified cosmetic compositions shown in Tables 1 to 4 were stored for 3 months in a thermostat at 45°C and 1 month at 60°C, and then evaluated with the naked eyes for the appearance thereof.

### [Judgment criteria for viscosity change]

A: No change or a slight change was found in the viscosity
B: Apparent viscosity change was found
C: Separated

### (3) Feeling upon use test

10 Special panelists used the oil-in-water emulsified cosmetic compositions shown in Tables 1 to 4 after the above (3) Long-term stability test (45°C, 3 months) was completed. The feeling upon use when applied was evaluated and judged for "absence of a friction feeling when applied," "absence of a sticky feeling after application" and "intensity of water repellency" based on the following evaluation criteria and judgment criteria. Those which had been separated in the long-term stability test were not evaluated.

### (Evaluation criteria)

5: Very favorable
4: Slightly favorable
3: Indeterminable
2: Slightly unfavorable
1: Very unfavorable

### (Judgment criteria)

A: Average point of 4.0 or more
B: Average point of 2.0 or more and less than 4.0
C: Average point of less than 2.0

**[Table 1]**

| Component (%) | | Comparative Examples | | | | Examples | | | |
|---|---|---|---|---|---|---|---|---|---|
| | | 1 | 2 | 3 | 4 | 1 | 2 | 3 | 4 |
| (A) | 2-(4-Diethylamino-2-hydroxybenzoyl)-benzoic acid hexyl ester | 1 | | | | 1 | 1 | | |
| | 2,4-Bis{[4-(2-ethylhexyloxy)-2-hydroxy]phenyl}-6-(4-methoxyphenyl)-1,3,5-triazine | | 1 | | | | | 1 | |
| | 2,4,6-Tris[4-(2-ethylhexyloxycarbonyl)anilino]-1,3,5-triazine | | | 1 | | | | | 1 |
| | 4-tert-Butyl-4'-methoxybenzoylmethane | | | | 1 | | | | |
| (B) | 2-Ethylhexyl paramethoxycinnamate | 10 | 10 | 10 | 10 | 10 | 10 | 10 | 10 |
| | Dimethicodiethyl benzal malonate | 0.5 | 0.5 | 0.5 | 0.5 | 0.5 | 0.5 | 0.5 | 0.5 |
| (Oil) | Octyldodecyl myristate | 3 | 3 | 3 | 3 | 3 | 3 | 3 | 3 |
| | Isohexadecane | 0.45 | 0.45 | 0.45 | 0.45 | 0.45 | 0.45 | 0.45 | 0.45 |
| (C) | Na acrylate/Na acryloyldimethyltaurate copolymer | 0.75 | 0.75 | 0.75 | 0.75 | 0.75 | 0.75 | 0.75 | 0.75 I |
| (D) | Silicone-surface treated fine particle zinc oxide powder (Production Example 1) | 5 | 5 | 5 | 5 | 10 | 20 | 10 | 10 |
| (E) | Ethanol | 15 | 15 | 15 | 15 | 15 | 15 | 15 | 15 |
| | POE (20) sorbitan monooleate | 0.15 | 0.15 | 0.15 | 0.15 | 0.15 | 0.15 | 0.15 | 0.15 |
| | Sorbitan oleate | 0.05 | 0.05 | 0.05 | 0.05 | 0.05 | 0.05 | 0.05 | 0.05 |
| | Purified water | Balance | Balance | Balance | Balance | Balance | Balance | Balance | Balance |
| Component (A) + Component (D) | | 6.00 | 6.00 | 6.00 | 5.00 | 11.00 | 21.00 | 11.00 | 11.00 |
| Amount of liquid oil | | 13.95 | 13.95 | 13.95 | 13.95 | 13.95 | 13.95 | 13.95 | 13.95 |
| (Component (A) + Component (D))/Amount of liquid oil | | 0.43 | 0.43 | 0.43 | 0.36 | 0.79 | 1.51 | 0.79 | 0.79 |
| Test results | | | | | | | | | |
| | UV protective ability (long-lasting effect) | C | C | C | C | A | A | A | A |
| | Emulsion state | A | A | A | A | A | A | A | A |
| | Crystallization state | A | A | A | C | A | A | A | A |
| | Long-term stability (60°C, 1 month) | A | A | A | B | A | A | A | A |
| | (45°C, 3 months) | B | B | B | B | A | A | A | A |

**[Table 2]**

| Component (%) | | Comparative Examples | | | | |
|---|---|---|---|---|---|---|
| | | 5 | 6 | 7 | 8 | 9 |
| (A) | 2-(4-Diethylamino-2-hydroxybenzoyl)-benzoic acid hexyl ester | | 1 | | | |
| | 2,4-Bis{[4-(2-ethylhexyloxy)-2-hydroxy]phenyl}-6-(4-methoxyphenyl)-1,3,5-triazine | | | 1 | | |
| | 2,4,6-Tris[4-(2-ethylhexyloxycarbonyl)anilino]-1,3,5-triazine | | | | 1 | |
| | 4-tert-Butyl-4'-methoxybenzoylmethane | 1 | | | | 1 |
| (B) | 2-Ethylhexyl paramethoxycinnamate | 10 | 10 | 10 | 10 | 10 |
| | Dimethicodiethyl benzal malonate | 0.5 | 0.5 | 0.5 | 0.5 | 0.5 |
| (Oil) | Octyldodecyl myristate | 3 | 3 | 3 | 3 | 3 |
| | Isohexadecane | 0.45 | 0.45 | 0.45 | 0.45 | 0.45 |
| (C) | Na acrylate/Na acryloyldimethyltaurate copolymer | 0.75 | 0.75 | 0.75 | 0.75 | 0.75 |
| (D) | Silicone-surface treated fine particle zinc oxide powder (Production Example 1) | 10 | 30 | 30 | 30 | 30 |
| (E) | Ethanol | 15 | 15 | 15 | 15 | 15 |
| | POE (20) sorbitan monooleate | 0.15 | 0.15 | 0.15 | 0.15 | 0.15 |
| | Sorbitan oleate | 0.05 | 0.05 | 0.05 | 0.05 | 0.05 |
| | Purified water | Balance | Balance | Balance | Balance | Balance |
| Component (A) + Component (D) | | 10.00 | 31.00 | 31.00 | 31.00 | 30.00 |
| Amount of liquid oil | | 13.95 | 13.95 | 13.95 | 13.95 | 13.95 |
| (Component (A) + Component (D))/Amount of liquid oil | | 0.72 | 2.22 | 2.22 | 2.22 | 2.15 |
| Test results | | | | | | |
| | UV protective ability (long-lasting effect) | A | - | - | - | - |
| | Emulsion state | A | B | B | B | B |
| | Crystallization state | C | A | A | A | C |
| | Long-term stability (60°C, 1 month) | B | C | C | C | C |
| | (45°C, 3 months) | B | C | C | C | C |

**[Table 3]**

| Component (%) | | Examples | | | | | | | | | | | | | | | | |
|---|---|---|---|---|---|---|---|---|---|---|---|---|---|---|---|---|---|---|
| | | 5 | 6 | 7 | 8 | 9 | 10 | 11 | 12 | 13 | 14 | 15 | 16 | 17 | 18 | 19 | 20 | 21 |
| (A) | 2-(4-Diethylamino-2-hydroxybenzoyl)-benzoic acid hexyl ester | 2 | 2 | 2 | 2 | 2 | 2 | 2 | 2 | 2 | 2 | 2 | 2 | 3 | 2 | 2 | 2 | 2 |
| | 2,4-Bis{[4-(2-ethylhexyloxy)-2-hydroxy]phenyl} -6-(4-methoxyphenyl)-1,3,5-triazine | - | - | - | - | - | - | 1 | - | - | - | - | - | - | - | - | - | - |
| | 2-Ethylhexyl dimethoxybenzylidene dioxoimidazolidine propionate | - | - | - | - | - | - | 1 | - | - | - | - | - | - | - | - | - | - |
| (B) | 2-Ethylhexyl paramethoxycinnamate | 10 | 10 | 10 | 10 | 10 | 10 | 12 | 10 | 10 | 10 | 10 | 10 | 8 | 10 | 10 | 10 | ' 10 |
| | Octocrylene | - | - | - | - | - | - | 4 | - | - | - | - | - | - | - | - | - | - |
| | Dimethicodiethyl benzal malonate | - | - | - | - | - | - | 1 | - | - | - | - | - | - | - | - | - | - |
| (Oil) | Octyldodecyl myristate | 3 | 3 | 3 | 3 | - | - | - | 3 | 3 | 3 | 3 | 3 | 10 | 3 | 3 | 3 | 3 |
| | Isononyl isononanoate | - | - | - | - | 3 | - | - | - | - | - | - | - | - | - | - | - | - |
| | Diisopropyl sebacate | - | - | - | - | - | 1 | - | - | - | - | - | - | - | - | - | - | - |
| | Glyceryl tri(2-ethylhexanoate) | - | - | - | - | - | 1 | - | - | - | - | - | - | - | - | - | - | - |
| | Propylene glycol isostearate | - | - | - | - | - | 1 | - | - | - | - | - | - | - | - | - | - | - |
| | Isohexadecane | 0.45 | 0.9 | - | - | 0.45 | 0.45 | 0.45 | 0.45 | 0.45 | 0.45 | 0.45 | 0.45 | 0.45 | 0.45 | 0.45 | 0.45 | 0.45 |
| | Squalane | - | - | 0.51 | - | - | - | - | - | - | - | - | - | - | - | - | - | - |
| | Hydrogenated polyisobutene | - | - | - | 0.72 | - | - | - | - | - | - | - | - | - | - | - | - | - |
| (C) | Na acrylate/Na acryloyldimethyltaurate copolymer | 0.75 | 1.5 | - | 0.75 | 0.75 | 0.75 | 0.75 | 0.75 | 0.75 | 0.75 | 0.75 | 0.75 | 0.75 | 0.75 | 0.75 | 0.75 | 0.75 |
| | Hydroxyethyl acrylate/Na acryloyldimethyltaurate copolymer | - | - | 0.75 | - | - | - | - | - | - | - | - | - | - | - | - | - | - |
| | Polyacrylamide | - | - | - | 1.2 | - | - | - | - | - | - | - | - | - | - | - | - | - |
| | Carboxy vinyl polymer K | - | - | - | - | - | - | - | - | - | - | - | - | - | - | - | - | - |
| | Xanthan gum | - | - | - | - | - | - | - | - | - | - | - | - | - | - | - | - | - |
| (D) | Silicone-surface treated fine particle zinc oxide powder (Production Example 1) | 15 | 15 | 15 | 15 | 15 | 15 | 15 | - | - | - | - | 10 | 10 | 15 | 15 | 15 | 15 |
| | Methyl hydrogen polysiloxane-surface treated fine particle zinc oxide powder (Production Example 2) | - | - | - | - | - | - | - | 20 | 8 | - | - | - | - | - | - | - | - |
| | Octyltriethoxysilane-surface treated fine particle zinc oxide powder (Production Example 4) | - | - | - | - | - | - | - | - | - | 15 | - | - | - | - | - | - | - |
| | Octyltriethoxysilane-surface treated flaky powder of zinc oxide (Production Example 5) | - | - | - | - | - | - | - | - | - | - | 15 | - | - | - | - | - | - |
| | Methyl hydrogen polysiloxane-surface treated fine particle titanium dioxide powder production Example 3) | - | - | - | - | - | - | - | - | - | - | - | 5 | - | - | - | - | - |
| | Zinc oxide | - | - | - | - | - | - | - | - | - | - | - | - | - | - | - | - | - |
| | Titanium dioxide | - | - | - | - | - | - | - | - | - | - | - | - | - | - | - | - | - |
| (E) | Ethanol | 15 | 15 | 15 | 15 | 15 | 15 | 15 | 15 | 15 | 15 | 15 | 15 | 15 | - | 5 | - | - |
| | 1,3-Butylene glycol | - | - | - | - | - | - | - | - | - | - | - | - | - | - | - | 15 | |
| | Propylene glycol | - | - | - | - | - | - | - | - | - | - | - | - | - | - | - | - | 15 |
| | POE (20) sorbitan monooleate | 0.15 | 0.3 | - | - | 0.15 | 0.15 | 0.15 | 0.15 | 0.15 | 0.15 | 0.15 | 0.15 | 0.15 | 0.15 | 0.15 | 0.15 | 0.15 |
| | Sorbitan oleate | 0.05 | 0.1 | - | - | 0.05 | 0.05 | 0.05 | 0.05 | 0.05 | 0.05 | 0.05 | 0.05 | 0.05 | 0.05 | 0.05 | 0.05 | 0.05 |
| | POE (20) Sorbitan monostearate | - | - | 0.11 | - | - | - | - | - | - | - | - | - | - | - | - | - | - |
| | Sorbitan isostearate | - | - | 0.03 | - | - | - | - | - | - | - | - | - | - | - | - | - | - |
| | Polyoxyethylene (7) lauryl ether | - | - | - | 0.18 | - | - | - | - | - | - | - | - | - | - | - | - | - |
| | Potassium hydroxide | - | - | - | - | - | - | - | - | - | - | - | - | - | - | - | - | - |
| | Purified water | Balance | Balance | Balance | Balance | Balance | Balance | Balanc e | Balanc e | Balanc e | Balanc e | Balanc e | Balance | Balanc e | Balanc e | Balanc e | Balance | Balanc e |
| Component (A) + Component (D) | | 17.00 | 17.00 | 17.00 | 17.00 | 17.00 | 17.00 | 19.00 | 22.00 | 10.00 | 17.00 | 17.00 | 12.00 | 13.00 | 17.00 | 17.00 | 17.00 | 17.00 |
| Amount of liquid oil | | 13.45 | 13.90 | 13.51 | 13.72 | 13.45 | 13.45 | 17.45 | 13.45 | 13.45 | 13.45 | 13.45 | 13.45 | 18.45 | 13.45 | 13.45 | 13.45 | 13.45 |
| (Component (A) + Component (D))/Amount of liquid oil | | 1.26 | 1.22 | 1.26 | 1.24 | 1.26 | 1.26 | 1.09 | 1.64 | 0.74 | 1.26 | 1.26 | 0.89 | 0.70 | 1.26 | 1.26 | 1.26 | 1.26 |
| Test results | | | | | | | | | | | | | | | | | | |
| | UV protective ability (long-lastinp effect) | A | A | A | A | A | A | A | A | C | A | A | A | C | A | A | A | A |
| | Emulsion state | A | A | A | A | A | A | A | A | A | A | B | A | A | B | B | B | B |
| | Crystallization state | A | A | A | A | A | A | A | A | A | A | A | A | A | A | A | A | A |
| | Long-term stability (60°C, 1 month) | A | A | A | A | A | A | A | A | A | A | B | A | A | B | B | B | B |
| | (45°C, 3 months) | A | A | B | B | A | A | A | A | A | A | B | A | A | B | B | B | B |
| | Feeling upon use (Absence of friction feeling) | A | A | A | A | A | A | A | A | A | B | A | A | A | A | A | A | A |
| | (Absence of sticky feeling) | A | B | A | A | A | A | A | A | A | A | B | A | B | B | B | B | B |
| | (Water repellency) | A | A | A | A | A | A | A | A | B | A | A | A | A | A | A | A | A |

**[Table 4]**

| Component (%) | | Comparative Examples | | | | | |
|---|---|---|---|---|---|---|---|
| | | 10 | 11 | 12 | 13 | 16 | 17 |
| (A) | 2-(4-Diethylamino-2-hydroxybenzoyl)-benzoic acid hexyl ester | 2 | 2 | 2 | 2 | 10 | 2 |
| | 2,4-Bis{[4-(2-ethylhexyloxy)-2-hydroxy]phenyl}-6-(4-methoxyphenyl)-1,3,5-triazine | - | - | - | - | - | - |
| | 2-Ethylhexyl dimethoxybenzylidene dioxoimidazolidine propionate | - | - | - | - | - | - |
| (B) | 2-Ethylhexyl paramethoxycinnamate | 10 | 10 | 10 | 10 | 10 | 10 |
| | Octocrylene | - | - | - | - | - | - |
| | Dimethicodiethyl benzal malonate | - | - | - | - | - | - |
| (Oil) | Octyldodecyl myristate | 3 | 3 | 3 | 3 | 3 | 10 |
| | Isononyl isononanoate | - | - | - | - | - | - |
| | Diisopropyl sebacate | - | - | - | - | - | - |
| | Glyceryl tri(2-ethylhexanoate) | - | - | - | - | - | - |
| | Propylene glycol isostearate | - | - | - | - | - | - |
| | Isohexadecane | 0.45 | 0.45 | 0.45 | 0.45 | 0.45 | 0.45 |
| | Squalane | - | - | - | - | - | - |
| | Hydrogenated polyisobutene | - | - | - | - | - | - |
| (C) | Na acrylate/Na acryloyldimethyltaurate copolymer | - | - | - | 0.75 | 0.75 | 0.75 |
| | Hydroxyethyl acrylate/Na acryloyldimethyltaurate copolymer | - | - | - | - | - | - |
| | Polyacrylamide | - | - | - | - | - | - |
| | Acrylamide/ammonium acrylate copolymer | - | - | - | - | - | - |
| | Polyacrylate-13 | - | - | - | - | - | - |
| | Carboxy vinyl polymer K | - | 0.4 | - | - | - | - |
| | Xanthan gum | - | - | 0.3 | - | - | - |
| (D) | Silicone-surface treated fine particle zinc oxide powder (Production Example 1) | 15 | 15 | 15 | - | 20 | 10 |
| | Methyl hydrogen polysiloxane-surface treated fine particle zinc oxide powder (Production Example 2) | - | - | - | - | - | - |
| | Octyltriethoxysilane-surface treated fine particle zinc oxide powder (Production Example 4) | - | - | - | - | - | - |
| | Octyltriethoxysilane-surface treated flakypowder of zinc oxide (Production Example 5) | - | - | - | - | - | - |
| | Methyl hydrogen polysiloxane-surface treated fine particle titanium dioxide powder (Production Example 3) | - | - | - | - | - | - |
| | Zinc oxide | - | - | - | 15 | - | - |
| | Titanium dioxide | - | - | - | - | - | - |
| (E) | Ethanol | 15 | 15 | 15 | 15 | 15 | 15 |
| | 1,3-Butylene glycol | - | - | - | - | - | - |
| | Propylene glycol | - | - | - | - | - | - |
| | POE (20) sorbitan monooleate | - | - | - | 0.15 | 0.15 | 0.15 |
| | Sorbitan oleate | - | - | - | 0.05 | 0.05 | 0.05 |
| | POE (20) sorbitan monostearate | - | - | - | - | - | - |
| | Sorbitan isostearate | - | - | - | - | - | - |
| | Polyoxyethylene (7) lauryl ether | - | - | - | - | - | - |
| | Potassium hydroxide | - | 0.2 | - | - | - | - |
| | Purified water | Balance | Balance | Balance | Balance | Balance | Balance |
| Component (A) + Component (D) | | 17.00 | 17.00 | 17.00 | 2.00 | 30.00 | 12.00 |
| Amount of liquid oil | | 13.45 | 13.45 | 13.45 | 13.45 | 13.45 | 20.45 |
| (Component (A) + Component (D))/Amount of liquid oil | | 1.26 | 1.26 | 1.26 | 0.15 | 2.23 | 0.59 |
| Test results | | | | | | | |
| | UV protective ability (long-lasting effect) | - | - | - | - | - | A |
| | Emulsion state | C | C | C | C | C | B |
| | Crystallization state | - | - | - | - | - | A |
| | Long-term stability (60°C, 1 month) | C | C | C | C | C | B |
| | (45°C, 3 months) | C | C | C | C | C | A |
| | Feeling upon use (Absence of friction feeling) | - | - | - | - | - | A |
| | (Absence of sticky feeling) | - | - | - | - | - | C |
| | (Water repellency) | - | - | - | - | - | A |

The hydrophobized powders used in the present Examples, Comparative Examples and Formulation Examples are as follows.

### (Production Example 1 Preparation of silicone-surface treated fine particle zinc oxide powder)

88 Parts by weight of a fine particle zinc oxide powder (near-spherical, average primary particle diameter of 0.016 µm) and 12 parts by weight of dimethyl polysiloxane (20 cSt, manufactured by Shin-Etsu Chemical Co., Ltd.) were put in toluene and vigorously stirred and then toluene was removed by heating under reduced pressure. The obtained powder was crushed using an atomizer. Subsequently, the calcination treatment was carried out at 800°C for 2 hours in the air using a high temperature heating furnace to obtain a silicone-surface treated fine particle zinc oxide powder.

### (Production Example 2 Preparation of methyl hydrogen polysiloxane-surface treated fine particle zinc oxide powder)

A slurry composed of 93 parts by mass of a fine particle zinc oxide powder (near-spherical, average particle diameter of 0.02 µm), 7 parts by mass of methyl hydrogen polysiloxane (KF-99P, manufactured by Shin-Etsu Chemical Co., Ltd.) and isopropyl alcohol was prepared, vigorously stirred and crushed, then the solvent was distilled off by heating under reduced pressure, and heat treatment was carried out at 150°C for 4 hours in the air to obtain a methyl hydrogen polysiloxane-surface treated fine particle zinc oxide powder.

### (Production Example 3 Preparation of methyl hydrogen polysiloxane-surface treated fine particle titanium dioxide powder)

A slurry composed of 95 parts by mass of a fine particle titanium dioxide powder (near-spherical, average particle diameter of 0.017 µm), 5 parts by mass of methyl hydrogen polysiloxane (KF-99P, manufactured by Shin-Etsu Chemical Co., Ltd.) and isopropyl alcohol was prepared, vigorously stirred and crushed, then the solvent was distilled off by heating under reduced pressure, and heat treatment was carried out at 160°C for 4 hours in the air to obtain a methyl hydrogen polysiloxane-surface treated fine particle titanium dioxide powder.

### (Production Example 4 Preparation of octyltriethoxysilane-surface treated fine particle zinc oxide powder)

A slurry composed of 93 parts by mass of a fine particle zinc oxide powder (near-spherical, average particle diameter of 0.02 µm), 7 parts by mass of octyltriethoxysilane and toluene was prepared, and crushed and pulverized using a bead mill (DYNO-MILL, manufactured by Shinmaru Enterprises Corporation). Subsequently, toluene was distilled off by heating under reduced pressure and then the heat treatment was carried out at 150°C for 4 hours using an air blast stream-type dryer to obtain an octyltriethoxysilane-surface treated fine particle zinc oxide powder.

### (Production Example 5 Preparation of octyltriethoxysilane-surface treated flaky powder of zinc oxide)

A slurry composed of 93 parts by mass of a flaky powder of zinc oxide (average particle diameter of 0.3 µm, average particle thickness of 0.032 µm, plate ratio of 9, iron element content of 0.01 mol%), 7 parts by mass of octyltriethoxysilane and toluene was prepared, and crushed and pulverized using a bead mill (DYNO-MILL, manufactured by Shinmaru Enterprises Corporation). Subsequently, toluene was distilled off by heating under reduced pressure and then the heat treatment was carried out at 150°C for 4 hours using an air blast stream-type dryer to obtain an octyltriethoxysilane-surface treated flaky powder of zinc oxide.

The results shown in Tables 1 to 4 reveal that the cosmetic composition of the present invention, when compared with Comparative Examples, has excellent UV protective ability and long-term stability, and further has an excellent feeling upon use. Particularly, from the comparison of Examples with Comparative Examples 1 to 4, 6 to 8, and 13 to 17, it is apparently difficult that both of the UV protective ability and stability are achieved simultaneously unless both of (i) the content of the component (A) and the component (D) and (ii) the content ratio of the component (A) and the component (D) relative to the liquid oil are within the range of the present invention. Further, in Comparative Examples 4, 5 and 9, it was found that 4-tert-butyl-4'-methoxybenzoylmethane, which is a solid organic ultraviolet absorber, was recrystallized in the inner phase and failed to exhibit a sufficient ultraviolet protective ability. In the test results, the samples marked with the "-" represent those having poor stability and thus not evaluated.

Formulation Examples of the oil-in-water emulsified cosmetic compositions of the present invention are shown below.

### Formulation Examples 1 to 3 (Makeup bases)

The oil-in-water emulsified cosmetic compositions (makeup bases) having the following formulations were prepared and evaluated, and as a result, were excellent in all of the ultraviolet protective effect, the feeling upon use and the storage stability.

| (Component blended) | | (% by mass) | |
|---|---|---|---|
| | Ex. 1 | Ex. 2 | Ex. 3 |
| 1. Octyltriethoxysilane-surface treated fine particle zinc oxide powder (Production Example 4) | | | |
| | 8.0 | 8.0 | 8.0 |
| 2. Methyl hydrogen polysiloxane-surface treated fine particle zinc oxide powder (Production Example 2) | | | |
| | 7.0 | 7.0 | 7.0 |
| 3. Diethylamino hydroxybenzoyl hexyl benzoate | | | |
| | 2.0 | 2.0 | 2.0 |
| 4. 2-Ethylhexyl paramethoxycinnamate | | | |
| | 10.0 | 10.0 | 10.0 |
| 5. Octyldodecyl myristate | 2.0 | 2.0 | 2.0 |
| 6. Sorbitan stearate | 0.3 | 0.3 | 0.3 |
| 7. Polyoxyethylene-Methyl polysiloxane copolymer | | | |
| (Silicone KF-6017, Shin-Etsu Chemical Co., Ltd.) | | | |
| | 0.3 | 0.3 | 0.3 |
| 8. Dimethicone (6cs) | 3.0 | 3.0 | 3.0 |
| 9. Squalane | 3.0 | 3.0 | 3.0 |
| 10. Polymethylsilsesquioxane | 3.0 | 3.0 | 3.0 |
| (Average primary particle diameter of 4.5 µm) | | | |
| 11. Ethanol | 10.0 | 10.0 | 10.0 |
| 12. Na acrylate/Acryloyldimethyl taurate copolymer | | | |
| (SIMULGEL EG, manufactured by SEPPIC) | 2.5 | - | - |
| 13. Acrylamide/Ammonium acrylate copolymer | | | |
| (SEPIPLUS 265, manufactured by SEPPIC) | - | 1.2 | - |
| 14. Hydroxyethyl acrylate/Na acryloyldimethyl taurate copolymer | | | |
| (SEPIGEL FL, manufactured by SEPPIC) | - | - | 3.0 |
| 15. Disodium edetate | 0.02 | 0.02 | 0.02 |
| 16. Glycerin | 5.0 | 5.0 | 5.0 |
| 17. 1,3-Butylene glycol | 5.0 | 5.0 | 5.0 |
| 18. Acerola extract | 1.0 | 1.0 | 1.0 |
| (Trade name: Acerola extract BG25 (manufactured by Maruzen Pharmaceuticals Co., Ltd.)) | | | |
| 19. Prune extract | 0.5 | 0.5 | 0.5 |
| (Trade name: Prune extract WC (manufactured by Maruzen Pharmaceuticals Co., Ltd.)) | | | |
| 20. Hyaluronic Acid | 0.5 | 0.5 | 0.5 |
| 21. Fragrance | 0.1 | 0.1 | 0.1 |
| 22. Phenoxy ethanol | 0.3 | 0.3 | 0.3 |
| 23. Purified water | Balance | Balance | Balance |

### Formulation Examples 4 to 6 (Sunscreens)

The oil-in-water emulsified cosmetic compositions (sunscreens) having the following formulations were prepared and evaluated, and as a result, were excellent in all of the ultraviolet protective effect, the feeling upon use and the storage stability.

| (Component blended) | | (% by mass) | |
|---|---|---|---|
| | Ex. 4 | Ex. 5 | Ex. 6 |
| 1. Octyltriethoxysilane-surface treated flaky powder of zinc oxide | | | |
| (Production Example 5) | | | |
| | 3.0 | 3.0 | 3.0 |
| 2. Methyl hydrogen polysiloxane-surface treated fine particle zinc oxide powder (Production Example 2) | | | |
| | 9.0 | 9.0 | 9.0 |
| 3. Diethylamino hydroxybenzoyl hexyl benzoate | | | |
| | 1.0 | 1.0 | 1.0 |
| 4. 2,4-Bis{[4-(2-ethylhexyloxy)-2-hydroxy]phenyl}-6-(4-methoxyphenyl)-1,3,5-triazine | | | |
| | 1.0 | 1.0 | 1.0 |
| 5. 2-Ethylhexyl paramethoxycinnamate | | | |
| | 10.0 | 10.0 | 10.0 |
| 6. Diisopropyl sebacate | 5.0 | 5.0 | 5.0 |
| 7. Octyldodecyl myristate | 3.0 | 3.0 | 3.0 |
| 8. Ethanol | 15.0 | 15.0 | 15.0 |
| 9. Ammonium polyacrylate | | | |
| (SIMULGEL A, manufactured by SEPPIC) | 3.0 | - | - |
| 10. Polyacrylate-13 | | | |
| (SEPIPLUS 400, manufactured by SEPPIC) | - | 1.4 | - |
| 11. Hydroxyethyl acrylate/Na acryloyldimethyl taurate copolymer | | | |
| (SEPIPLUS S, manufactured by SEPPIC) | - | - | 1.7 |
| 12. Disodium edetate | 0.02 | 0.02 | 0.02 |
| 13. 1,3-Butylene glycol | 5.0 | 5.0 | 5.0 |
| 14. Dipropylene glycol | 2.0 | 2.0 | 2.0 |
| 15. Fragrance | 0.1 | 0.1 | 0.1 |
| 16. Phenoxy ethanol | 0.1 | 0.1 | 0.1 |
| 17. Purified water | Balance | Balance | Balance |

The formulation of the fragrances used in the above Formulation Examples is shown in Table 5.

**[Table 5]**

| Fragrance formulation | | | |
|---|---|---|---|
| Component | ‰ by mass | Component | ‰ by mass |
| Terpineol | 10.00 | Vanillin | 2.00 |
| Terpinyl acetate | 2.00 | Ethyl vanillin | 0.10 |
| Cepionate | 60.00 | Muscone | 0.50 |
| Methyl dihydrojasmonate | 250.00 | Ethylene brassylate | 42.00 |
| Indole | 0.05 | 4,6,6,7,8,8-Hexamethyl-1,3,4,6,7,8-hexahydrocyclopentabenzopyran | 60.00 |
| 2-Methyl-3-(3,4-methylenedioxy-phenyl)-propanal | 3.00 | Cyclopentadecanolide | 20.00 |
| Hydroxycitronellal | 20.00 | Ambrettolide | 1.00 |
| Hydroxycitronellol | 10.00 | γ-Undecalactone | 0.40 |
| p-t-Butyl-α-methylhydrocinnamic aldehyde | 35.00 | γ-Decalactone | 0.10 |
| 4-(4-Hydroxy-4-methyl-pentyl)-3-cyclohexene-1-carboxaldehyde | 75.00 | 4-(4-Hydroxyphenyl)-2-butanone | 0.50 |
| 3-Methyl-5-phenylpentanol | 20.00 | Musk ketone | 0.10 |
| Phenyl ethyl alcohol | 10.00 | Skatole | 0.01 |
| α-Ionone | 10.00 | cis-Jasmon | 0.05 |
| β-Ionone | 20.00 | Phenylethyl acetate | 0.10 |
| γ-Methyl ionone | 10.00 | Civetone | 0.20 |
| Dihydro-β-ionone | 25.00 | γ-Nonalactone | 0.05 |
| Benzyl salicylate | 150.00 | α-Santalol | 0.20 |
| Cis-3-hexenyl salicylate | 30.00 | β-Santalol | 0.20 |
| Eugenol | 0.80 | Eugenyl acetate | 0.10 |
| Cinnamic alcohol | 5.00 | α-Hexyl cinnamic aldehyde | 20.00 |
| Cinnamic aldehyde | 0.50 | α-Damascone | 0.04 |
| Guaiol acetate | 1.00 | β-Damascone | 0.02 |
| Guaiol | 0.50 | β-Damascenone | 0.01 |
| Cedrenyl acetate | 5.00 | δ-Damascone | 0.01 |
| Cedryl methyl ketone | 30.00 | Rose absolute | 0.50 |
| 6,7-Dihydro-1,1,2,3,3-pentamethyl-4(5H)-indane | 2.00 | Rose oil | 4.50 |
| Vetiver acetate | 10.00 | Sandalwood oil | 2.00 |
| 3-Methyl-5-(2,3,3-trimethyl-3-cyclopenten-1-yl)-pentan-2-ol | 2.00 | Labdanum absolute | 0.05 |
| 2-Ethyl-4-(2,3,3-trimethyl-3-cyclopenten-1-yl)-2-buten-1-ol | 0.80 | Cisto absolute | 0.01 |
| Isobornyl cyclohexanol | 35.00 | Vetiver oil | 0.50 |
| Heliotropin | 10.00 | Guaiac wood oil | 0.10 |
| Coumarin | 2.00 | Total | 1000.00 |

## Claims

1. An oil-in-water emulsified cosmetic composition comprising the following components (A) to (D), and satisfying the following conditions (i) and (ii):
[Components]
(A) at least one selected from the group consisting of 2-(4-diethylamino-2-hydroxybenzoyl)benzoic acid hexyl ester, 2,4-bis{[4-(2-ethylhexyloxy)-2-hydroxy]phenyl}-6-(4-methoxyphenyl)-1,3,5-triazine, 2,4,6-tris[4-(2-ethylhexyloxycarbonyl)anilino]-1,3,5-triazine and 2-ethylhexyl dimethoxybenzylidene dioxoimidazolidine propionate,
(B) 8% by mass or more and 20% by mass or less of a liquid oil including a liquid organic ultraviolet absorber, the mass content ratio of the liquid organic ultraviolet absorber in the liquid oil being 0.5 or more, wherein the liquid organic ultraviolet absorber is at least one selected from 2-ethylhexyl paramethoxycinnamate, octocrylene and dimethicodiethyl benzal malonate,
(C) a polyacrylamide compound, and
(D) a hydrophobized fine particle metal oxide powder;
[Conditions]
(i) a total content of the component (A) and the component (D) ((A) + (D)) relative to a total amount of the cosmetic composition is 10% by mass or more, and
(ii) a content mass ratio of the total content of the component (A) and the component (D) ((A) + (D)) relative to the total amount of the liquid oil (component(B)) is 0.6 or more and 2 or less.

2. The oil-in-water emulsified cosmetic composition according to claim 1, wherein the total content of the component (A) and the component (D) ((A) + (D)) is 10 to 28% by mass.

3. The oil-in-water emulsified cosmetic composition according to claim 1 or 2, further comprising (E) a saturated monohydric alcohol having 1 to 3 carbon atoms.

4. The oil-in-water emulsified cosmetic composition according to any one of claims 1 to 3, comprising 8 to 22% by mass of the component (E) in a total amount of the cosmetic composition.

5. The oil-in-water emulsified cosmetic composition according to any one of claims 1 to 4, comprising 0.1 to 2% by mass of the component (C), relative to a total amount of the cosmetic composition.

6. The oil-in-water emulsified cosmetic composition according to any one of claims 1 to 5, comprising 0 to 3% by mass of a surfactant.

7. The oil-in-water emulsified cosmetic composition according to any one of claims 1 to 6, comprising 10 to 20% by mass of the component (B) in a total amount of the cosmetic composition.

8. The oil-in-water emulsified cosmetic composition according to any one of claims 1 to 7, wherein the component (C) is at least one selected from the group consisting of a polyacrylamide and a copolymer containing acrylamide and/or acryloyldimethyl taurate as a constituent unit.

9. The oil-in-water emulsified cosmetic composition according to any one of claims 1 to 8, wherein the metal oxide of the component (D) is one or two or more selected from the group consisting of zinc oxide, titanium dioxide and cerium oxide.

10. The oil-in-water emulsified cosmetic composition according to any one of claims 1 to 9, wherein the average particle diameter of the component (D) is 0.01 to 1 µm.

11. The oil-in-water emulsified cosmetic composition according to any one of claims 1 to 10, wherein a hydrophobization treatment of the component (D) is at least one surface treatment selected from the group consisting of fluorine compound treatment, silicone treatment, silicone resin treatment, pendant treatment, silane coupling agent treatment, polyacrylic acid treatment, metallic soap treatment, amino acid treatment, inorganic compound treatment, plasma treatment, mechanochemical treatment, and silane compound or silazane compound treatment.

12. The oil-in-water emulsified cosmetic composition according to any one of claims 1 to 11, wherein the hydrophobization treatment of the component (D) is at least one surface treatment selected from the group consisting of a surface treatment using silicone or a silicone resin, a surface treatment using methyl hydrogen polysiloxane, and a surface treatment using a silane compound or silazane compound.

13. The oil-in-water emulsified cosmetic composition according to any one of claims 1 to 12, wherein the hydrophobization treatment of the component (D) is at least one surface treatment selected from the group consisting of a surface treatment using silicone or a silicone resin and a surface treatment using methyl hydrogen polysiloxane.

14. Composition for use according to any one of claims 1 to 13 as a sunscreen cosmetic composition.

## Patentansprüche

1. Öl-in-Wasser-emulgierte kosmetische Zusammensetzung, die die folgenden Komponenten (A) bis (D) umfasst und die folgenden Bedingungen (i) und (ii) erfüllt:
[Komponenten]
(A) mindestens einen Vertreter, ausgewählt aus der Gruppe, bestehend aus 2-(4-Diethylamino-2-hydroxybenzoyl)benzoesäurehexylester, 2,4-Bis{[4-(2-ethylhexyloxy)-2-hydroxy]phenyl}-6-(4-methoxyphenyl)-1,3,5-triazin, 2,4,6-Tris[4-(2-ethylhexyloxycarbonyl)anilinyl]-1,3,5-triazin und 2-Ethylhexyldimethoxybenzylidendioxoimidazolidinpropionat,
(B) 8 Massen-% oder mehr und 20 Massen-% oder weniger eines flüssigen Öls, das einen flüssigen organischen UV-Absorber enthält, wobei das Massengehaltsverhältnis des flüssigen organischen UV-Absorbers in dem flüssigen Öl 0,5 oder mehr beträgt, wobei der flüssige organische UV-Absorber mindestens ein Vertreter, ausgewählt aus 2-Ethylhexylparamethoxycinnamat, Octocrylen und Dimethicodiethylbenzalmalonat, ist
(C) eine Polyacrylamidverbindung, und
(D) ein hydrophobiertes Feinpartikel-Metalloxidpulver;
[Bedingungen]
(i) der Gesamtgehalt der Komponente (A) und der Komponente (D) ((A) + (D)) beträgt bezogen auf die Gesamtmenge der kosmetischen Zusammensetzung 10 Massen-% oder mehr, und
(ii) das Gehaltsmassenverhältnis des Gesamtgehalts der Komponente (A) und der Komponente (D) ((A) + (D)) beträgt bezogen auf die Gesamtmenge des flüssigen Öls (Komponente (B)) 0,6 oder mehr und 2 oder weniger.

2. Öl-in-Wasser-emulgierte kosmetische Zusammensetzung gemäß Anspruch 1, wobei der Gesamtgehalt der Komponente (A) und der Komponente (D) ((A) + (D)) 10 bis 28 Massen-% beträgt.

3. Öl-in-Wasser-emulgierte kosmetische Zusammensetzung gemäß Anspruch 1 oder 2, zusätzlich umfassend einen gesättigten einwertigen Alkohol (E) mit 1 bis 3 Kohlenstoffatomen.

4. Öl-in-Wasser-emulgierte kosmetische Zusammensetzung gemäß einem der Ansprüche 1 bis 3, umfassend 8 bis 22 Massen-% der Komponente (E) in der Gesamtmenge der kosmetischen Zusammensetzung.

5. Öl-in-Wasser-emulgierte kosmetische Zusammensetzung gemäß einem der Ansprüche 1 bis 4, umfassend 0,1 bis 2 Massen-% der Komponente (C), bezogen auf eine Gesamtmenge der kosmetischen Zusammensetzung.

6. Öl-in-Wasser-emulgierte kosmetische Zusammensetzung gemäß einem der Ansprüche 1 bis 5, umfassend 0 bis 3 Massen-% eines Tensids.

7. Öl-in-Wasser-emulgierte kosmetische Zusammensetzung gemäß einem der Ansprüche 1 bis 6, umfassend 10 bis 20 Massen-% der Komponente (B) in der Gesamtmenge der kosmetischen Zusammensetzung.

8. Öl-in-Wasser-emulgierte kosmetische Zusammensetzung gemäß einem der Ansprüche 1 bis 7, wobei die Komponente (C) mindestens ein Vertreter, ausgewählt aus der Gruppe, bestehend aus einem Polyacrylamid und einem Copolymer, das Acrylamid und/oder Acryloyldimethyltaurat als Struktureinheit enthält, ist.

9. Öl-in-wasser-emulgierte kosmetische Zusammensetzung gemäß einem der Ansprüche 1 bis 8, wobei das Metalloxid der Komponente (D) ein oder zwei oder mehr Vertreter, ausgewählt aus der Gruppe, bestehend aus Zinkoxid, Titandioxid und Ceroxid, ist.

10. Öl-in-Wasser-emulgierte kosmetische Zusammensetzung gemäß einem der Ansprüche 1 bis 9, wobei der mittlere Partikeldurchmesser der Komponente (D) 0,01 bis 1 µm beträgt.

11. Öl-in-Wasser-emulgierte kosmetische Zusammensetzung gemäß einem der Ansprüche 1 bis 10, wobei eine Hydrophobierungsbehandlung der Komponente (D) mindestens eine Oberflächenbehandlung ist, die aus der Gruppe ausgewählt ist, die aus einer Behandlung mit einer Fluorverbindung, einer Silikonbehandlung, einer Silikonharzbehandlung, einer Pendant-Behandlung (pendant treatment), einer Behandlung mit einem Silan-Kupplungsmittel, einer Polyacrylsäurebehandlung, einer Behandlung mit einer Metallseife, einer Aminosäurebehandlung, einer Behandlung mit einer anorganischen Verbindung, einer Plasmabehandlung, einer mechanochemischen Behandlung und einer Behandlung mit einer Silanverbindung oder einer Silazanverbindung besteht.

12. Öl-in-Wasser-emulgierte kosmetische Zusammensetzung nach einem der Ansprüche 1 bis 11, wobei die Hydrophobierungsbehandlung der Komponente (D) mindestens eine Oberflächenbehandlung ist, die aus der Gruppe ausgewählt ist, die aus einer Oberflächenbehandlung unter Verwendung von Silikon oder eines Silikonharzes, einer Oberflächenbehandlung unter Verwendung von Methylhydrogenpolysiloxan und einer Oberflächenbehandlung unter Verwendung einer Silanverbindung oder einer Silazanverbindung besteht.

13. Öl-in-Wasser-emulgierte kosmetische Zusammensetzung gemäß einem der Ansprüche 1 bis 12, wobei die Hydrophobierungsbehandlung der Komponente (D) mindestens eine Oberflächenbehandlung ist, die aus der Gruppe ausgewählt ist, die aus einer Oberflächenbehandlung unter Verwendung von Silikon oder eines Silikonharzes und einer Oberflächenbehandlung unter Verwendung von Methylhydrogenpolysiloxan besteht.

14. Zusammensetzung gemäß einem der Ansprüche 1 bis 13 zur Verwendung als eine Sonnenschutz-Kosmetikzusammensetzung.

## Revendications

1. Composition cosmétique en émulsion huile-dans-eau comprenant les composants (A) à (D) suivants, et satisfaisant aux conditions (i) et (ii) suivantes :
[Composants]
(A) au moins un sélectionné dans le groupe constitué de l'ester hexylique d'acide 2-(4-diéthylamino-2-hydroxybenzoyl)benzoïque, de la 2,4-bis{[4-(2-éthylhexyloxy)-2-hydroxy]phényl}-6-(4-méthoxyphényl)-1,3,5-triazine, de la 2,4,6-tris[4-(2-éthylhexyloxycarbonyl)anilino]-1,3,5-triazine et du propionate de 2-éthylhexyle diméthoxybenzylidène dioxoimidazolidine,
(B) 8% en masse ou plus et 20% en masse ou moins d'une huile liquide incluant un absorbeur ultraviolet organique liquide, le rapport de la teneur en masse de l'absorbeur ultraviolet organique liquide dans l'huile liquide étant 0,5 ou plus, dans laquelle l'absorbeur ultraviolet organique liquide est au moins l'un sélectionné parmi le 2-éthylhexyle paraméthoxycinnamate, l'octocrylène et le diméthicone diéthyle benzal malonate,
(C) un composé polyacrylamide, et
(D) une poudre de particules d'oxyde métallique fines rendue hydrophobe ;
[Conditions]
(i) une teneur totale en composant (A) et en composant (D) ((A) + (D)) par rapport à une quantité totale de la composition cosmétique est 10 % en masse ou plus, et
(ii) un rapport massique de teneur de la teneur totale en composant (A) et en composant (D) ((A) + (D)) par rapport à la quantité totale de l'huile liquide (composant (B)) est 0,6 ou plus et 2 ou moins.

2. Composition cosmétique en émulsion huile-dans-eau selon la revendication 1, dans laquelle la teneur totale en composant (A) et en composant (D) ((A) + (D)) est 10 à 28 % en masse.

3. Composition cosmétique en émulsion huile-dans-eau selon la revendication 1 ou 2, comprenant en outre (E) un alcool monohydrique saturé ayant 1 à 3 atomes de carbone.

4. Composition cosmétique en émulsion huile-dans-eau selon l'une quelconque des revendications 1 à 3, comprenant 8 à 22 % en masse du composant (E) dans une quantité totale de la composition cosmétique.

5. Composition cosmétique en émulsion huile-dans-eau selon l'une quelconque des revendications 1 à 4, comprenant 0,1 à 2 % en masse du composant (C), par rapport à une quantité totale de la composition cosmétique.

6. Composition cosmétique en émulsion huile-dans-eau selon l'une quelconque des revendications 1 à 5, comprenant 0 à 3 % en masse d'un tensioactif.

7. Composition cosmétique en émulsion huile-dans-eau selon l'une quelconque des revendications 1 à 6, comprenant 10 à 20 % en masse du composant (B) dans une quantité totale de la composition cosmétique.

8. Composition cosmétique en émulsion huile-dans-eau selon l'une quelconque des revendications 1 à 7, dans laquelle le composant (C) est au moins l'un sélectionné dans le groupe constitué d'un polyacrylamide et d'un copolymère contenant de l'acrylamide et/ou de l'acryloyldiméthyle taurate en tant qu'unité constituante.

9. Composition cosmétique en émulsion huile-dans-eau selon l'une quelconque des revendications 1 à 8, dans laquelle l'oxyde métallique du composant (D) est un ou deux ou plus sélectionnés dans le groupe constitué d'oxyde de zinc, de dioxyde de titane et d'oxyde de cérium.

10. Composition cosmétique en émulsion huile-dans-eau selon l'une quelconque des revendications 1 à 9, dans laquelle le diamètre de particule moyen du composant (D) est 0,01 à 1 µm.

11. Composition cosmétique en émulsion huile-dans-eau selon l'une quelconque des revendications 1 à 10, dans laquelle un traitement d'hydrophobisation du composant (D) est au moins un traitement de surface sélectionné dans le groupe constitué d'un traitement par un composé de fluor, d'un traitement par de la silicone, d'un traitement par une résine de silicone, d'un traitement par chaîne latérale, d'un traitement par un agent adhésif au silane, d'un traitement par de l'acide polyacrylique, d'un traitement par un savon métallique, d'un traitement par un acide aminé, d'un traitement par un composé inorganique, d'un traitement par un plasma, d'un traitement mécano-chimique, et d'un traitement par un composé silane ou par un composé silazane.

12. Composition cosmétique en émulsion huile-dans-eau selon l'une quelconque des revendications 1 à 11, dans laquelle le traitement d'hydrophobisation du composant (D) est au moins un traitement de surface sélectionné dans le groupe constitué d'un traitement de surface utilisant de la silicone ou une résine de silicone, d'un traitement de surface utilisant un polysiloxane hydrogéné méthylique, et un traitement de surface utilisant un composé silane ou un composé silazane.

13. Composition cosmétique en émulsion huile-dans-eau selon l'une quelconque des revendications 1 à 12, dans laquelle le traitement d'hydrophobisation du composant (D) est au moins un traitement de surface sélectionné dans le groupe constitué d'un traitement de surface utilisant de la silicone ou une résine de silicone et un traitement de surface utilisant un polysiloxane hydrogéné méthylique.

14. Composition à utiliser selon l'une quelconque des revendications 1 à 13 en tant que composition cosmétique de crème solaire.
